(19) Europäisches Patentamt
European Patent Office
Office européen des brevets

(11) Publication number: 0 373 508 A2

# (12) EUROPEAN PATENT APPLICATION

(21) Application number: 89122573.2

(22) Date of filing: 07.12.89

(51) Int. Cl.5: C07D 493/10, G01N 33/532, G01N 33/542, G01N 33/94, //(C07D493/10,311:00,307:00)

(30) Priority: 12.12.88 US 284781

(43) Date of publication of application: 20.06.90 Bulletin 90/25

(84) Designated Contracting States: AT BE CH DE ES FR GB IT LI NL

(71) Applicant: ABBOTT LABORATORIES
One Abbott Park Road
Abbott Park, IL 60064-3500(US)

(72) Inventor: Adamczyk, Maciej Bogdan
2015 Sprucewood Lane
Lindenhurst Illinois 60046(US)
Inventor: Dubler, Robert Edward
860 Greenleaf
Gurnee Illinois 60031(US)
Inventor: Cantarero, Luis Augusto
1319 Dunleer
Mundelein Illinois 60060(US)
Inventor: Jonas, Patrick Francis
1608 Alexander Court
Waukegan Illinois 60085(US)

(74) Representative: Modiano, Guido et al
MODIANO, JOSIF, PISANTY & STAUB
Modiano & Associati Via Meravigli, 16
I-20123 Milano(IT)

## (54) Barbiturate assay, tracers, immunogens and antibodies.

(57) The present invention is directed to a fluorescence polarization immunoassay for barbiturates, to the various components needed for preparing and carrying out such an assay, and to methods of making these components. Specifically, tracers, immunogens and antibodies are disclosed, as well as methods for preparing them. The tracers and the immunogens are made from substituted barbiturate compounds. A fluorescein moiety is included in the tracer, while a poly(amino acid) forms a part of the immunogen. The assay is conducted by measuring the degree of polarization retention of plane-polarized light that has been passed through a sample containing antiserum and tracer.

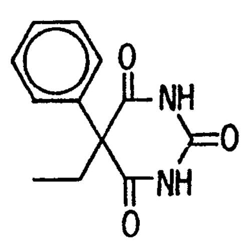

FIG. 1-1

EP 0 373 508 A2

Xerox Copy Centre

# BARBITURATE ASSAY, TRACERS, IMMUNOGENS AND ANTIBODIES

## BACKGROUND OF THE INVENTION

### Technical Field

The present invention relates to a method and reagents for a fluorescence polarization immunoassay procedure for determining the amount of barbiturate in fluids, especially biological fluids such as serum, plasma or urine, and to a method of making the reagents. More specifically, the invention relates to (1) reagents (tracers and antibodies) for determining the amount of barbiturate in a sample; (2) immunogen compounds used to raise antibodies; (3) synthetic methods (for making the tracer and immunogen compounds); and (4) analytical methods for conducting the assay.

### Background Art

Barbiturates are central nervous system depressants. Therapeutically, they are used as sedatives, hypnotics and anticonvulsants. Although the legal availability of barbiturates has declined, they are frequently abused sedative or hypnotic drugs and are commonly used to commit suicide.

The physiological absorption, action and toxicity of barbiturates vary widely and are dependent on the nature of the 5-substituted groups and imino-hydrogens. Approximately 35% of the barbiturate in blood is plasma protein bound. Barbiturates are distributed in various tissues and organs. Barbiturates are primarily metabolized in the liver and, with a few exceptions, are generally excreted in urine mainly as nonactive metabolites.

The most commonly abused barbiturates are the short to medium acting: secobarbital, pentobarbital, amobarbital, etc. These are widely used to reduce excitation states due to the use of stimulants. Tolerance to these drugs can develop from chronic use, and death may occur from either overdose or abrupt withdrawal of the drug.

In the past, barbiturate levels in urine have typically been measured by high performance liquid chromatography (HPLC), gas chromatography (GC), enzyme immunoassay (EIA), substrate-linked fluorescence immunoassay (SLFIA) and radioimmunoassay (RIA). These methods are reasonably specific for detecting drug levels; however, they are not without drawbacks. HPLC and GC methods require sample extraction procedures and the assay time is lengthy. Both EIA and SLFIA involve enzyme reactions and have the following disadvantages: 1) the reagents are relatively unstable; 2) any components in the biological samples which may influence the enzyme reaction in EIA or SLFIA (such as enzyme inhibitors or enzymes which catalyze similar reactions) will affect the assay results; and 3) EIA and SLFIA measure either absorbance or fluorescence, and any compounds in the biological samples which may affect absorbance or fluorescence (such as lipid, hemoglobin, bilirubin or other chromophores or fluorophores) will affect the accuracy of the results obtained from these assays. RIA reagents have the following shortcomings: 1) short shelf-life; 2) radiation hazards; and 3) problems associated with the storage and disposal of radioactive materials.

In assays for drugs and other substances, fluorescence polarization competitive binding immunoassays have provided a more satisfactory alternative. Typically, competitive binding immunoassays are used for measuring ligands in a test sample. (For purposes of this disclosure, a "ligand" is a substance of biological interest to be determined quantitatively by a competitive binding immunoassay technique.) The ligands compete with a labeled reagent, or "ligand analog," or "tracer," for a limited number of binding sites on antibodies specific to the ligand and ligand analog. The concentration of ligand in the sample determines the amount of ligand analog which binds to the antibody: the amount of ligand analog that will bind is inversely proportional to the concentration of ligand in the sample, because the ligand and the ligand analog each bind to the antibody in proportion to their respective concentrations.

Fluorescence polarization provides a quantitative means for measuring the amount of tracer-antibody conjugate produced in a competitive binding immunoassay. Fluorescence polarization techniques are based on the principle that a fluorescent labeled compound, when excited by plane-polarized light, will emit fluorescence having a degree of polarization inversely related to its rate of molecular rotation. Accordingly, when a tracer-antibody conjugate having a fluorescent label is excited with plane-polarized light, the emitted light remains highly polarized because the fluorophore is constrained by the antibody from rotating between

the time that light is absorbed and emitted. In contrast, when an unbound tracer is excited by plane-polarized light, its rotation is much faster than that of the corresponding tracer-antibody conjugate. As a result, the light emitted from the unbound tracer molecules is depolarized.

A problem that heretofore has prevented the accurate determination of barbiturates and other "drugs of abuse" in urine by fluorescence polarization techniques is that of riboflavin interference. Riboflavin, or vitamin $B_2$, is a common constituent of many foods and of commercially available vitamin supplements. Riboflavin is excreted primarily in the urine and has a fluorescence spectrum quite similar to that of fluorescein. As a result, the presence of riboflavin in even moderate amounts in urine samples creates an interference which can produce erroneous results. While ordinary consumption of riboflavin is unlikely to produce more than trace amounts of riboflavin in the urine, test results can readily be distorted by the consumption of excessive quantities of vitamin supplements by persons wishing to prevent detection of barbiturate use.

The present invention offers an advance in the art in that highly sensitive tracers, a method for making the tracers, and an assay using the tracers are provided specifically for the determination of barbiturates without riboflavin interference. Further, the invention is characterized by a more uniform cross-reactivity for the commonly used barbiturates.

## SUMMARY OF THE INVENTION

The present invention is directed to a fluorescence polarization assay for barbiturates; to tracers, immunogens and antibodies for use in the assay; and to methods for making the tracers, immunogens and antibodies.

A first aspect of the invention relates to the discovery of unique tracers and immunogens having novel structures. According to the first aspect of the invention the tracers and the immunogens can both be represented by the formula shown in Figure 2 wherein:

W is oxygen or sulfur:

$R_1$ is alkyl, alkenyl, or alkynyl having a total of from 1 to 12 carbon atoms arranged in a straight or branched chain and containing up to two aliphatic or aromatic ring structures;

$R_2$ is $CH_2$-R-Z-Q;

Q is a poly (amino acid), a poly(amino acid) derivative or another immunologically active carrier; or fluorescein or a fluorescein derivative;

Z is NH, CO, CS, $SO_2$ or C=NH when Q is fluorescein or a fluorescein derivative and CO, O-CONH, N, NH, N=N, or $CH_2$ when Q is a poly(amino acid) or poly(amino acid) derivative or another immunologically active carrier; and

R is a linking group having a total of from 0 to 20 carbon atoms and heteroatoms arranged in a straight or branched chain and containing up to two ring structures; and including up to 7 heteroatoms when Q is a poly(amino acid); a poly(amino acid) derivative or other immunologically active carrier; up to 10 heteroatoms when Q is fluorescein or a fluorescein derivative and the linking group includes no ring structures; and up to 12 heteroatoms when Q is fluorescein or a fluorescein derivative and the linking group includes one or two ring structures.

When Q is a poly(amino acid), a derivative thereof, or any other immunologically active carrier, the compound can be used as an immunogen. When Q is fluorescein or a derivative thereof, the compound can be used as a tracer.

In a preferred embodiment, the tracers and immunogens are represented by the structural Formula of Figure 2 wherein:

W, $R_2$ and Q are defined <u>supra</u>;

$R_1$ is alkyl having a total of from 2 to 7 carbon atoms arranged in a straight or branched chain and containing up to one aliphatic or aromatic ring structure;

Z is NH or CO when Q is fluorescein or a fluorescein derivative and $CH_2$ when Q is a poly(amino acid) or poly(amino acid) derivative or another immunologically active carrier; and

R is a linking group having a total of from 0 to 10 carbon atoms and heteroatoms arranged in a straight or branched chain and containing up to two ring structures; and including up to 4 heteroatoms when Q is a poly(amino acid), a poly(amino acid) derivative or other immunologically active carrier; up to 6 heteroatoms when Q is fluorescein or a fluorescein derivative and the linking group includes no ring structure; and up to 6 heteroatoms when Q is fluorescein or a fluorescein derivative and the linking group includes one or two aliphatic or aromatic ring structures.

A second aspect of the invention relates to antibodies raised by the novel immunogen. According to the

second aspect of the invention, antibodies are prepared in response to a compound according to claim 1 when Q is a poly(amino acid) or a derivative thereof or another immunologically active carrier.

According to a third aspect of the invention, an immunogen is made by a method comprising the step of coupling a compound represented by the structural formula shown in Figure 2 wherein:

W is oxygen or sulfur;

$R_1$ is alkyl, alkenyl or alkynyl having a total of from 1 to 12 carbon atoms arranged in a straight or branched chain and containing up to two aliphatic or aromatic ring structures;

$R_2$ is $CH_2$-R-X;

X is $NH_2$, Cl, Br, I, OH, $CO_2H$, O-C-Cl, - $\underset{O}{\underset{\|}{C}}$-$CH_2I$, -CHO, - $\underset{O}{\underset{\|}{C}}$-$CH_3$,

or

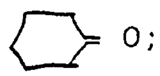

and

R is a linking group including up to 7 heteroatoms and having a total of from 0 to 20 carbon atoms and heteroatoms arranged in a straight or branched chain and containing up to two ring structures;

with a poly(amino acid), a poly(amino acid) derivative or another immunologically active carrier.

According to a fourth aspect of the invention, a method is provided for making a tracer by coupling a compound represented by the structural formula shown in Figure 2, wherein:

W is oxygen or sulfur;

$R_1$ is alkyl, alkenyl or alkynyl having a total of from 1 to 12 carbon atoms arranged in a straight or branched chain and containing up to two aliphatic or aromatic ring structures;

$R_2$ is $CH_2$-R-Y;

Y is - $NH_2$, COOH, -COCl, $SO_3H$; $SO_2Cl$, SH, CHO, CN, OH, or I; and

R is a linking group including up to 10 heteroatoms, having a total of from 0 to 20 carbon atoms and heteroatoms arranged in a straight or branched chain and containing up to two aliphatic or aromatic ring structures;

with fluorescein or a derivative of fluorescein.

Preferably, the tracer is prepared by coupling a precursor of the structural formula shown in Figure 2 wherein:

W and $R_2$ are defined supra;

$R_1$ is alkyl having 4 or 5 carbon atoms arranged in a branched chain and containing no ring structure;

Y is $NH_2$ or COOH; and

R is a linking group including up to 3 heteroatoms, having a total of from 3 to 5 carbon atoms and heteroatoms arranged in a straight or branched chain and containing no ring structure.

Preferred derivatives of fluorescein include amino, amido, amidino, urea, thiourea, carbamido, thiocarbamido or triazinylamino derivatives. Most preferred at the present time are the amino derivatives, particularly aminomethylfluorescein.

A fifth aspect of the invention relates to the elimination of potential fluorescence interference by riboflavin. Riboflavin binding protein (RBP) is added either directly to each sample or to one or more of the reagents utilized in the assay; wherein it binds all riboflavin present into RBP-riboflavin complexes, thus eliminating fluorescence interference. Other fluorescence-quenching substances may also be utilized for this purpose.

According to a sixth aspect of the invention, a process for detecting or measuring the concentration of barbiturates is provided. A sample is contacted with barbiturate antiserum, and a fluorescein-containing barbiturate derivative capable of producing a detectable fluorescence polarization response to the presence of the barbiturate antiserum. Plane-polarized light is then passed through the solution to obtain a fluorescence polarization response, and this response is detected as a measure of the amount of barbiturate in the sample.

Further objects and attendant advantages of the invention will be best understood from a reading of the following detailed description taken together with the Figures and the Examples.

## BRIEF DESCRIPTION OF THE DRAWINGS

In the following Figures the symbol "F1" represents a fluorescein moiety, and the various other symbols are noted in the Detailed Description.

Figure 1 shows the general structure of the barbiturates to be semi-quantitatively determined in accordance with the present invention.

Figure 2 shows a general structural formula for the tracers and the immunogens of the present invention as well as the classes of reactants used in preparing them.

Figure 3 shows the alternate structural Formulae and names of the fluorescein moiety included in the tracers of the present invention.

Figure 4 shows various linkages that couple the fluorescein moiety to the precursor in Figure 2, when Figure 2 represents a precursor for the tracers.

Figures 5 through 19 show various examples of structures of tracers in accordance with the present invention.

Figures 20 through 26 show various examples of structures of hapten reactants used to form the immunogens employed in the present invention.

## DETAILED DESCRIPTION OF THE INVENTION

The various aspects of the invention will now be discussed in detail in relation to the Figures.

The present invention involves the use of fluorescein and derivatives of fluorescein. In particular, a necessary property of fluorescein and its derivatives for the usefulness of the tracer compounds of the present invention is the fluorescence of fluorescein. Fluorescein exists in two tautomeric forms, illustrated in Figure 3, depending on the acid concentration (pH) of the environment. In the open (acid) form, there are a number of conjugated double bonds which make that form of fluorescein (and compounds containing a fluorescein moiety) capable of absorbing blue light and emitting green fluorescence after an excited state lifetime of about four nanoseconds. When the open and closed forms coexist, the relative concentration of molecules in the open and closed forms is easily altered by adjustment of the pH level. Generally, the tracer compounds of the present invention exist in solution as biologically acceptable salts such as sodium, potassium, ammonium and the like, allowing the compounds to exist in the open, fluorescent form when employed in the analytical methods of the present invention. The specific salt present will depend on the buffer employed to adjust the pH level. For example, in the presence of a sodium phosphate buffer, the compounds of the present invention will generally exist in the open form, as a sodium salt.

As used herein, the term "fluorescein," either as an individual compound or as a component of a larger compound, is meant to include both the open and closed forms, if they exist for a particular molecule, except in the context of fluorescence. An open form is necessary for the fluorescence to occur.

The numbering of carbon atoms of the fluorescein molecule varies, depending upon whether the open or closed form of the molecule is considered. Accordingly, the literature concerning fluorescein and its compounds is not uniform as to carbon atom numbering. In the closed form, the para-carbon to the carbonyl of the lactone on the phenyl ring is numbered 6. In the open form, the para-carbon to the carboxylic acid group on the phenyl ring is numbered 5 (see Figure 3). In this disclosure the numbering of the closed form is adopted because the raw materials used in the syntheses are most popularly numbered with that system. The carbon atom of fluorescein and its compounds which is opposite the carboxyl group is therefore numbered "6" for purposes of the present disclosure.

A tracer in solution which is not complexed to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes the rotation of the antibody molecule, which is slower than that of the relatively small tracer molecule, thereby increasing the polarization observed. Therefore, when a ligand competes with the tracer for antibody bites, the observed polarization of fluorescence of the resulting mixture of the free tracer and tracer-antibody complex assumes a value intermediate between that of the tracer and that of the tracer-antibody complex. If a sample contains a high concentration of the ligand, the observed polarization value is closer to that of the free tracer, i.e., low. If the test sample contains a low concentration of the ligand, the polarization value is closer to that of the bound tracer, i.e., high. By sequentially exciting the reaction mixture of an immunoassay with vertically and then horizontally polarized light and analyzing only the vertical component of the emitted light, the polarization of fluorescence in the reaction mixture may be accurately determined. The precise relationship between polarization and concentration of the ligand to be determined is established by measuring the polarization values of calibrators with known concentrations of ligand. The concentration of

the ligand in a sample can be extrapolated from a standard curve prepared in this manner.

The particular antibodies and tracers formed in accordance with this invention have been found to produce very good assays, as discussed infra.

## The Reagents

Both the immunogens and the tracers of the present invention can be represented by the general structural formula set forth in the Summary of the Invention, and illustrated in Figure 2.

The objective is to have competition between barbiturate and the tracer for the recognition sites of the antibody. Great variations in the structure of the haptens and tracers are allowed in achieving this goal. For the purpose of this invention, "haptens" are precursors of the immunogens, comprising generally a substituted barbiturate derivative bearing a group suitable for linking to an immunologically active carrier.

## The Structure of the Immunogens

Usable antibodies can be produced from a variety of barbiturate derivatives. Immunogens made from compounds functionalized at the 5 position on the ring can produce antibodies in animals; such antibodies are useful in a barbiturates assay according to the invention when combined with the appropriate tracer.

The immunogens of the present invention have the general structural formula shown in Figure 2, and in the preferred form of the invention, the immunogens are also derived from the general structural formula shown in Figure 2. The immunogens can be prepared by coupling a compound of the class shown in Figure 2 with a poly (amino acid) or a derivative of a poly(amino acid) or another immunologically active carrier, as will be discussed in the context of the synthetic method and the Examples below.

Although bovine serum albumin is the poly (amino acid) in this preferred form, it should be understood that various protein carriers can be employed, including albumins, serum proteins, e.g., globulins, ocular lens proteins, lipoproteins and the like. Illustrative protein carriers include, in addition to bovine serum albumin, keyhole limpet hemocyanin, egg ovalbumin, bovine gamma-globulin, thyroxine binding globulin, etc. Alternatively, synthetic poly(amino acids) having a sufficient number of available carboxylate groups such as aspartates can be employed, as can many other synthetic or natural polymeric materials bearing reactive functional groups. In addition, carbohydrates, yeasts, polysaccharides or any other substance that can be used as an immunological carrier can be conjugated to the hapten to produce an immunogen.

## The Structure of the Tracers

The possible variations in the structure of the tracers of the invention are even greater than the possible variations in the structure of the haptens thereof. The tracers of the present invention have the general structural formula shown in Figure 2. In a preferred form of the invention, the tracer has the structural formula shown in Figure 5.

The tracer is a barbiturate derivative that is linked to a fluorescein derivative by, for example, an amido, amidino, triazinylamino, carbamido, thiocarbamido, carbamoyl, thiocarbamoyl, or sulfonylcarbamoyl group, as shown in Figure 4. The tracers are prepared by linking the appropriate fluorescein derivative to a barbiturate derivative containing an amino, carboxylic acid, sulfonic acid, mercapto, hydroxy, imidate, hydrazide, isocyanate, thioisocyanate, chloroformate, chlorothioformate, carboxylic chloride, chlorosulfonylcarbamoyl, or the like group, as will be discussed in the context of the synthetic method and the Examples below.

By way of example, any of the following fluorescein derivatives can be used:

Fl-$CH_2$-$NH_2$ aminomethyl fluorescein

Fl-$CO_2H$ fluorescein amine

F1-$CO_2H$ carboxyfluorescein

F1-$NHCOCH_2I$ α-iodoacetamidofluorescein

Fl-$NHCOCH_2Br$ α-bromoacetamidofluorescein

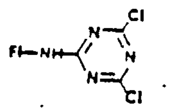

2,4-dichloro-1,3,5-triazin-2-ylamino-fluorescein (DTAF)

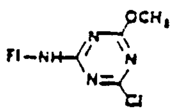

4-chloro-6-methoxy-1,3,5-triazin-2-ylamino-fluorescein

Fl-NCS fluorescein thioisocyanate

## The Antibodies

The antibodies of the present invention are prepared by eliciting a response in sheep to the immunogens described supra. The immunogen is administered to animals or to in vitro cultures of immunocompetent cells by a series of innoculations, in a manner well known to those skilled in the art. It should be understood that although sheep were the preferred immune host to barbiturate immunogens in the experiments detailed herein, any in vivo or in vitro host capable of producing antibodies to the structures herein outlined may be employed.

## The Synthesis of Immunogens

The immunogen of the present invention are made by coupling a hapten, such as shown by the general structure of Figure 2 when X is chloroformate, aldehyde, carboxylic, amino, chloride, bromide, iodide or hydroxy, to a poly(amino acid) or other immunologically active carrier. The poly(amino acid) or other carrier moiety can be linked to the hapten by a carbamate, amido, thioether, ether, diazo, or amino linkage. In a preferred embodiment, the poly(amino acid), is bovine serum albumin (BSA), and the hapten has the structure shown in Figure 20. These reactants are preferably coupled under conditions normally used to form carbamate linkages; such conditions are well known to those skilled in the art.

The immunogens are prepared by coupling a hapten that contains an aldehyde, carboxylic, amino, chlorine, bromine, iodine, hydroxide, or iodoacetonyl group to a poly(amino acid) or other immunologically active carrier. The aldehyde can be coupled by forming Schiff's base with poly(amino acid) or the amino group of another immunologically active carrier. Schiff's base is instantly reduced by sodium cyanoborohydride to form the stable aminomethyl linkage. The activation of the carboxylic groups on the poly(amino acid) can be accomplished by mixing the hapten and the poly(amino acid) with 1-ethyl-3-(3-dimethylamino-propyl) carbo-diimide (EDC), N,N′-dicyclohexylcarbodiimide (DCC), 1-cyclohexyl-3-(2-morpholinoethyl)carbodiimide; methyl-p-toluene sulfonate, or the like. The hydrazide case is coupled in the same manner as for the nonaromatic amino case. Alkyl, chloro, bromo, and iodo derivatives and sulfonate esters alkylate the phenolic hydroxyl groups of tyrosine residues in the carrier protein under strongly alkaline conditions to form alkyl aryl ethers, and alkylate the sulfur of free sulfhydryl groups cysteine to form thioethers. For these reactions the preferred derivatives are the iodoacetonyls and iodides.

The syntheses of the above haptens (immunogen precursors) are accomplished in one of two general ways. Figure 2 shows an immunogen precursor class in accordance with a preferred embodiment of the method of the present invention. The preparation proceeds from alkylating an appropriate substituted malonate or cyanoacetate ester with a bromoalkylalcohol having a protected alcohol functionality, preferably tetrahydropyranyl ether. The cyclization of such intermediate with urea can be accomplished by treating the mixture of the reactants in solution with a base such as magnesium methoxide, magnesium ethoxide, sodium methoxide, sodium ethoxide, or potassium t-butoxide. The protected functionality is revealed and the compound is coupled to the poly(amino acid) or other carrier.

5-iodoacetonyl barbiturate precursors of immunogens are prepared from appropriate 5,5-disubstituted barbiturates (as shown in Figure 2), bearing a 5-alkene group with a terminal double bond, by reacting with iodide in water. The 3-hydroxy-iodobarbiturates are prepared in this way. The corresponding α-iodoacetonyl barbiturates are obtained from these by oxidation with chromium-based oxidants such as Jones' reagent or

the like.

Aldehydes can be prepared from appropriate 5-alkenyl barbiturates (Figure 2) bearing a terminal double bond by ozonolysis in methanol at -78° C. Another preferred way to obtain aldehydes (Figure 2) is by reacting an appropriate substituted malonate ester or cyanoacetate ester with a bromoalkyl aldehyde having protected aldehyde functionality, preferably as acetals. The cyclization of such an intermediate with urea can be accomplished by treating the mixture of the reactants in solution with a base such as sodium ethoxide, sodium methoxide, potassium methoxide, or potassium t-butoxide. The protected functionality is revealed and the compound is coupled to the poly(amino acid) or other carrier.

Carboxylic acids are obtained by the oxidation of appropriate aldehydes with chromium-based oxidants such as Jones' reagent or the like.

A preferred way to synthesize carboxyalkyl barbiturates or carboxyalkenbarbiturates is by reacting appropriate 5-substituted barbiturates (Figure 2) with halogen alkyl esters or halogen alkenyl esters in the presence of bases such as triethylamine, sodium hydride, potassium t-butoxide, and followed by hydrolysis of appropriate barbiturate esters with a mineral acid such as concentrated hydrochloric acid, 40% sulfuric acid, or the like.

The alkyl halides may be prepared by treatment of the alcohols with hydrogen choride, hydrogen bromide or hydrogen iodide, or by treatment with halogenating reagents such as thionyl chloride. These halides couple directly to free sulfhydryl groups or carriers under relatively neutral conditions or to phenols such as those of tyrosyl residues in a poly(amino acid) under strongly basic conditions.

## The Synthesis of the Tracers

The tracers of the present invention are made by coupling a fluorescein moiety, or a derivative of fluorescein, to the general structure shown in Figure 2 wherein W i oxygen or sulfur; $R_1$ is alkyl, alkenyl or alkynyl having a total of from 1 to 12 carbon atoms arranged in a straight or branched chain and containing up to two aliphatic or aromatic ring structures; $R_2$ is $CH_2$-R-Y; R is a linking group having a total of from 0 to 20 carbon atoms and heteroatoms arranged on a straight or branched chain and containing up to two ring structures; and including up to 10 heteroatoms when the linking group includes no ring structures; and up to 12 heteroatoms when the linking group includes one or two ring structures; and Y is $-CO_2H$, -CHO, $SO_3H$, CN, OH, or $NH_2$.

The fluorescein moiety can be linked to the amino, carboxyl, aldehyde, acid chloride, imidate or alkoxy functional group by an amide, amine, urea, thiourea, carbamate, thiocarbamate, triazinylamino or sulfonylcarbamate linkage, as shown in Figure 4. In the presently preferred embodiment, the fluorescein derivative is aminomethylfluorescein, and this is coupled to a precursor shown in Figure 2, wherein $R_1$ is (methyl)butyl or 1-methyl propyl and $R_2$ is $-CH_2$, -COOH or $-CH_2-CH=CH-CO_2H$. The appropriate carboxyalkenyl barbiturate is coupled to aminomethylfluorescein by first forming the active ester with carboxylic acid of barbiturate. The preferred active ester is N-hydroxysuccinimide active ester and the preferred method is via N,N′-dicyclohexylcarbodiimide activation using anhydrous N,N-dimethyl formamide as a solvent.

Other activating groups, such as acid chloride, 1-hydroxybenzotriazole, p-nitrophenol, or 2-ethyl-5-phenylisoxazolium-3′-sulfonate can be used; and other solvents such as tetrahydrofuran, dimethylsulfoxide, hexamethylenephosphoramide can be used. The reactants are preferable coupled under conditions for forming amide linkages, and it is most preferred that active ester procedures be used. Usable tracers can be prepared from a variety of barbiturates.

All barbiturates that have a terminal amino group, such as amino, hydrazinyl, hydrazido, or the like, are coupled to carboxyfluorescein by the active ester method or the mixed anhydride method, and are coupled to fluorescein isothiocyanate, DTAF, or alkoxy DTAF simply by mixing the two materials in solution. The amino group can be converted to isocyanate or thioisocyanate groups by reaction with phosgene and thiophosgene respectively. These are then condensed with aminomethylfluorescein to produce the tracer.

All barbiturates that have a terminal aldehyde group are coupled to aminomethylfluorescein by reductive amination with sodium borohydride.

All barbiturates that have a terminal hydroxy group can be coupled to fluorescein by reaction with DTAF, α-iodoacetamidofluorescein, α-bromoacetamidofluorescein, or fluorescein isothiocyanate in solution. All barbiturates that have 5-iodoacetonyl group are coupled to aminomethylfluorescein to produce the tracer.

It should be noted that an aspect of the present invention encompasses the discovery that the presence of a carbon-carbon double bond between the fluorescein moiety and the barbiturate ring advantageously orients the tracer for optimal interaction with the antibody.

The Assay

The particular tracers and antibodies of the present invention have been found to produce surprisingly good results in fluorescence polarization assays for barbiturates. Figure 1 shows the general structure of the barbiturates to be quantitatively or qualitatively determined in accordance with the present invention. The assay of the present invention provides a more rapid and accurate barbiturates assay method than most prior art methods because it requires no specimen treatment before analysis and because of the broad-spectrum barbiturate specificity shown by the assay. The assay system accurately determines the presence of barbiturates in a sample, because antibody specificity precludes detection of virtually all compounds other than barbiturate-like compounds.

In accordance with the analytical methods of the present invention; i.e., the methods of determining barbiturates by a fluorescence immunoassay procedure using the tracer compounds and immunogens of the invention, a sample containing or suspected of containing barbiturates is intermixed with a biologically acceptable salt of a tracer and an antibody specific to barbiturates. The antibody is produced using the immunogen as described above. The barbiturates and the tracer compete for limited antibody binding sites, resulting in the formation of complexes. By maintaining constant the concentration of tracer and antibody, the ratio of barbiturates-antibody complex to tracer-antibody complex that is formed is directly proportional to the amount of barbiturates in the sample. Therefore, upon exciting the mixture with plane polarized light and measuring the polarization of the fluorescence emitted by a tracer and a tracer-antibody complex, one is able to determine the presence of barbiturates in the sample.

The results can be quantified in terms of net millipolarization units and span (in millipolarization units). The measurement of net millipolarization units indicates the maximum polarization when a maximum amount of the tracer is bound to the antibody, in the absence of any barbiturate. The higher the net millipolarization units, the better the binding of the tracer to the antibody. The span is the difference between the net millipolarization when tracer is maximally bound to the antibody in the absence of barbiturates and the net millipolarization when tracer is bound to antibody in the presence of specified concentration of barbiturate. The maximum span defines the range of ligand concentration which the assay is able to detect. A larger span provides for a better numerical analysis of data. The preferred antibody-tracer combination has a span of at least 80 millipolarization units. Smaller spans may be acceptable depending on the circumstances.

The pH at which the method of the present invention is practiced must be sufficient to allow the fluorescein moiety of the tracers to exist in their open form. The pH may range from about 3 to 12, more usually in the range of from about 5 to 10, most preferably from about 6 to 9. Various buffers may be used to achieve and maintain the pH during the assay procedure. Representative buffers include borate, phosphate, carbonate, tris, barbital and the like. The particular buffer employed is not critical to the present invention, but the tris and phosphate buffers are preferred. The cation portion of the buffer will generally determine the cation portion of the tracer salt in solution.

Riboflavin binding protein (RBP) may be added to the sample or to one or more of the assay reagents in order to bind any riboflavin present in the sample into RBP-riboflavin complexes, thus eliminating potential fluorescence interference front riboflavin. RBP is a protein of approximately 32,000 M.W. which is commonly isolated from egg whites. Upon isolation from the egg, each molecule of RBP contains one molecule of riboflavin. This, the holoprotein form of RBP, must be converted to the apoprotein form by dialysis, under acidic conditions, to remove the bound riboflavin. The RBP apoprotein utilized in the present invention is commercially available from Sigma Chemical Company, St. Louis, Missouri. The amount used is not critical, provided a sufficient quantity is used to bind virtually all free riboflavin in the sample.

The preferred method of the improved assay of the present invention will now be discussed in detail. The assay is a "homogeneous assay," which means that the end polarization readings are taken from a solution in which bound tracer is not separated from unbound tracer. This is a distinct advantage over heterogeneous immunoassay procedures such as those where the bound tracer must be separated from the unbound tracer before a reading can be taken.

The reagents for the fluorescence polarization assay of the present invention comprise antibody for barbiturates and barbiturate tracer. Additionally, largely conventional solutions including a pretreatment solution, a dilution buffer, barbiturate calibrators and barbiturates controls are desirably prepared. Typical solutions of these reagents, some of which are described below, are commercially available in assay "kits" from Abbott Laboratories, Abbott Park, Illinois.

All percentages expressed herein are weight/volume unless otherwise indicated. The tracer formulation presently preferred is 164 nanomolar tracer in: 0.1 molar phosphate buffer at pH 6.2; 5% sodium 5-sulfosalicylate; 0.1% sodium azide; and 0.01% bovine gamma-globulin. The antiserum formulation comprises sheep serum diluted with: 0.1 molar tris buffer at pH 7.5; 0.1% sodium azide; 0.1% bovine gamma-globulin; and 2% ethylene glycol (volume/volume). The dilution buffer comprises: 0.1 molar sodium phosphate at pH 7.5; 0.1% sodium azide; and 0.01% bovine gamma-globulin. The pretreatment solution comprises: 0.01% bovine gamma-globulin; 0.1 molar tris buffer at pH 7.5; 0.1% sodium azide; and 3mg/ml riboflavin binding protein. Barbiturate calibrators comprising secobarbital in normal human urine at concentrations of 0.0, 0.20, 0.40, 0.70, 1.20 and 2.00 micrograms per milliliter, with 0.1% sodium azide as a preservative, are useful. Barbiturate controls comprising secobarbital in normal human urine are provided at concentrations of 0.30 and 1.00 micrograms per milliliter with 0.1% sodium azide as a preservative are also useful.

The preferred procedure is especially designed to be used in conjunction with the Abbott TDx$^R$ polarization analyzer available from Abbott Laboratories, Irving, Texas. A minimum of fifty microliters of urine is required. The calibrators, controls, or unknown samples are pipetted directly into the sanple well of the TDx$^R$ sample cartridge. One of the advantages of this procedure is that the sample does not require any special preparation. The assay procedure from this point is fully automated.

If a manual assay is being performed, then the sample is mixed with the pretreatment solution in dilution buffer and a background reading is taken. The tracer and antibody are then mixed into the test solution. After incubation, a fluorescence polarization reading is taken.

The net fluorescence polarization value of each calibrator, control or sample is determined and is printed on the output tape of an instrument such as the Abbott TDx$^R$ Analyzer. A standard curve has previously been generated in the instrument by plotting the polarization of each calibrator versus its concentration using a nonlinear regression analysis. The concentration of each control or sample is read off the stored calibration curve and printed on the output tape.

With respect to the foregoing preferred procedure, it should be noted that the tracer, antibody, pretreatment solution, calibrators and controls should be stored between about 2°C and about 8°C while the dilution buffer should be stored at ambient temperature. A standard curve and controls should be run every two weeks, with each calibrator and control run in duplicate. All samples can be run in duplicate.

It should be understood that the foregoing detailed description and the following Examples are intended to be illustrative, but not limiting, with respect to the scope of the present invention. Various modifications will become apparent to one skilled in the art, and thus it is intended that the scope of the invention be defined solely by the claims and legal equivalents thereof.

## EXAMPLES

Examples 1 through 3 described experiments that were performed in accordance with the concepts of the present invention. Examples 1 through 3 are directed to the preparation of immunogens useful for producing antibodies; Examples 4 through 8 are directed to the synthesis of precursors for immunogens and tracers; and Examples 9 though 23 are directed to the preparation of tracers.

### Example 1

#### 5-(1-methylbutyl)-5-(β-hydroxy-γ-iodopropyl) barbituric acid

5 g (0.022 mol) of 5-(1-methylbutyl)-5-(allyl-barbituric acid in 180 ml of water was heated under a reflux condenser to 75-85°C. 6 g of iodide was added to the reaction mixture in small portions over a period of 2 hours. The reaction mixture was stirred and heated for 7 hours. The reaction mixture was cooled down and precipitated. Crystals were filtered off on a Büchner funnel and washed with water. The product was crystallized from ethanol (50 ml) to yield 5.57 g of the desired material.

#### 5-(1-methylbutyl)-5-iodoacetonyl barbituric acid

1 g of 5-(1-methylbutyl)-5-(β-hydroxy-γ-iodopropyl)barbituric acid was dissolved in 75 ml of acetone. 30 ml of a 0.33 mol solution of potassium dichromite in 10% sulfuric acid was added. The reaction mixture was stirred for 2 hours at room temperature and subsequently extracted with 200 ml of ethyl acetate, washed with brine, water and the organic layer was dried over magnesium sulfate. Ethy acetate was removed in vacuo and crude solid material crystallized from 70% ethanol. The yield was 519 mg of colorless crystals of the desired product.

Coupling of 5-(1-methyl)iodoacetonyl barbituric acid to bovine serum albumin

226 mg of BSA was dissolved in 4.7 ml of phosphate (buffer 0.1 M, pH 8) and 520 ul of N,N′-dimethylformamide. To this solution was added 152 mg of 5-(1-methylbutyl)-5-iodoacetonyl barbituric acid in 530 ul of DMF. The reaction mixture was stirred at room temperature for 36 hours. The resulting solution was dialysed exhaustively versus water and liophilized to give 208 mg of the desired conjugate.

Example 2

1-bromoethyl tetrahydropyranylether

To 2-bromoethanol (125 g, 1.0 equiv), cooled in an ice bath, was added p-toluenesulfonic acid monohydrate (catalytic amount). Dihydropyran (93 g, 1.1 equiv) was added dropwise, with stirring, under an argon atmosphere. When the addition was complete, the reaction was stirred for 1 hour at room temperature. The residue was purified by short path distillation, giving approximately 125 g of product.

Phenyl-tetrahydropyranyloxyethyl diethyl malonate

To diethyl phenylmalonate (76 ml, 1.0 equiv) in DMF was added NaH (13.1 g, 1.0 equiv, 64.14% mineral oil dispersion). The reaction was stirred for 1/2 hour at 50 °C then 1-bromo-2-tetrahydropyranylethanol (80 ml, 1.5 equiv) was added and the reaction stirred at 60 °C for 18 hours. The reaction mixture was neutralized and the solvent removed under reduced pressure. The residue was purified by short path distillation, giving about 46 g of product.

THP-hydroxyethylphenobarbital

Magnesium turnings (3.7 g, 1.2 equiv) were dissolved in dry methanol under an argon atmosphere. The THP-malonate (46 g, 1.0 equiv) and urea (9.9 g, 1.3 equiv) were dissolved together in dry methanol and then added to the magnesium solution. After refluxing for 48 hours, additional urea (9.9 g, 1.3 equiv) and magnesium methoxide (3.7 g of Mg, 1.2 equiv) were added. After refluxing an additional 24 hours, the solvent was removed under reduced pressure and the residue taken up in 5% aqueous HCl, then extracted with ether. The product was removed from the ether layer and crystallized from ether/hexane, giving about 13 g of material.

5-phenyl-5-hydroxyethyl barbituric acid

THP-hydroxyethylphenobarbital (19 g) was dissolved in acetic acid:water (50/50) with heat. The solution was refluxed and the deprotection monitored by TLC (silica gel, 10:90, methanol:methylene chloride). When the reaction was complete, the solvent was removed under reduced pressure and the residue crystallized

from ethanol: water. The yield was 8.7 grams of the product as a white powder.

### Conjugation of 5-phenyl-5-hydroxyethyl barbituric acid to BSA

Hydroxyethylphenylbarbital (500 mg) was suspended in dry, Freshly distilled THF (10 ml). Phosgene was bubbled into the solution with stirring for 15 minutes, following which argon was bubbled into the solution to remove the excess phosgene. After 15 minutes, the THF was removed under reduced pressure and the residue triturated with ethyl ether. After drying, the yield was about 520 mg of the product as a white powder.

To asolution of BSA (500 mg, 1.0 equiv) in 0.1 N, (pH 8) phosphate buffer (7 ml) was added first DMF (3 ml) and then the chloroformate of hydroxyethyl phenobarbital (50 mg) which had been dissolved in THF (1 ml). The chloroformate was added with vigorous stirring, and the reaction was stirred for an additional 3 hours.

The solution was purified using a PID column, eluting with distilled water Two peaks were collected, the first containing 100 mg of product and the second containing 400 mg of product. Examination by ultraviolet light indicated that both samples contained protein and phenobarbital.

## Example 3

### Diethyl-2-(1-methylbutyl)malonate

Sodium metal (5.24 g, 0.23 g atm) was reacted with 25 ml of absolute ethanol. To this solution was added dropwise, with stirring, diethyl malonate (36.0 ml, 0.24 mol). The solution was heated to reflux and 2-bromopentane (29.0 ml, 0.23 mol) was added dropwise. After refluxing overnight, the reaction was cooled and the ethanol removed on a rotovap. The product was washed with water, dried with $MgSO_4$ and fractionally distilled to give 35.7g of the desired product.

### Diethyl-2-(1-methylbutyl)-2-(5-pentenyl)-malonate

Potassium metal (1.71 g., 0.04 g atm) was reacted with 30 ml of anhydrous t-butyl alcohol. The reaction was then heated to 75° C and diethyl 2-(1-methylbutyl)-malonate (10.03 g, 0.04 mol) was added dropwise. After refluxing for four hours, 1-bromo-5-pentene (6.4 g, 0.04 mol) was added dropwise with stirring. The reaction was refluxed overnight and was then poured into water and extracted with ether. The ether extracts were dried with $MgSO_4$ and the ether evaporated on a rotovap to give the desired material.

### 5-(1-methylbutyl)-5-(pentenyl)-barbituric acid

Sodium metal (0.98 g, 0.04 g atm) was reacted with methanol (15 ml) followed by the addition of urea (5.14 g, 0.09 mol). Diethyl 2-(1-methyl-butyl)-2-(5-pentenyl)-malonate (6.08 g, 0.02 mol) was then added. After refluxing for two days, the methanol was distilled off. The residue was dissolved in 1N sodium hydroxide solution, extracted with ether and then made acidic with hydrochloric acid to give a white precipitate of the barbituric acid.

### 5-(4-butanal)-5-(1-methylbutyl)-barbituric acid

Ozone was passed through a solution of the above barbiturate in methanol at -78° C. After the solution

had turned blue, nitrogen was bubbled in to remove excess ozone. Dimethyl sulfide was then added and the solution stirred overnight at room temperature. The solvents were then removed under vacuum to give 0.62 g of the desired aldehyde.

Conjugation of the above aldehyde to BSA

To a solution of 25 ml of water, 5 ml of dimethylformamide and 5 ml of ethanol was added 188 mg of BSA. The pH of the solution was adjusted to 6.2 and the above aldehyde added (18.6 mg, 0.07 mmol). After stirring for one hour, sodium cyanoborohydride (472 mg, 7.5 mmol) was added and the reaction stirred overnight. The material was then dialyzed against water with a pH of 9 and then against water with a pH of 7. The material was then freeze dried to give 113 mg of the barbituric acid - BSA conjugate.

## Example 4

5-(1-methylbutyl)-5-formylmethyl barbituric acid

To a solution of sodium secobarbital (5.30 g, 22.2 mmol) in methanol was passed a stream of ozone until the solution turned a blue color. After excess ozone was removed by passing nitrogen through the reaction dimethyl sulfide (14 ml) was added. The reaction was stirred at room temperature overnight before solvents were removed on a rotovap. The aldehyde was then purified on silica prep plates using ethyl acetate:hexanes (1:1).

## Example 5

Dimethyl-2-(3-methylcyclohexyl)-malonate

Sodium metal (7.02 g, 0.30 g atm) was reacted with anhydrous methanol (250 ml). To this was then added dimethyl malonate (70 ml) dropwise while the temperature of the reaction was maintained at 50 °C. To this solution was then added dropwise 3-methylcyclohexylbromide (54.0 g, 0.30 mol) and the reaction refluxed overnight. The methanol was removed on a rotovap and the remaining material partitioned between ether and water. The ether layer was separated, dried with $MgSO_4$ and evaporated to give a yellow oil. Distillation of the material at 95-106 °C and 1.2 mm pressure gave a clear oil.

Dimethyl-2-(5-hexenyl)-2-(3-methylcyclohexyl)-malonate

To a slurry of sodium hydride (2.53 g, 0.06 mol) in anhydrous N,N-dimethylformamide (50 ml) was added dropwise dimethyl 2-(3-methylcyclohexyl)-malonate (14.46 g, 0.06 mol). After complete addition, the reaction was stirred until no more hydrogen gas was evolved. To the reaction was then added dropwise 1-bromo-6-hexene (10.33 g, 0.06 mol) followed by stirring. The progress of the reaction was followed by analytical chromatography using silica plates and ethyl acetate: hexanes (40:60). After complete reaction, water (20 ml) was added and most of the solvents removed under high vacuum. The remaining material was partitioned between water and ether and the ether layer washed twice with water. The ether solution was dried with $MgSO_4$ and evaporated to give a thick yellow oil. The product was then distilled at 127-134 °C and 0.4 mm pressure to give a clear oil.

5-(3-methylcyclohexyl)-5-(5-hexenyl)barbituric acid

Sodium metal (1.24 g, 0.05 g atm) was reacted with absolute ethanol (75 ml). After the reaction was complete, urea (5.16 g, 0.08 mol) was added followed by the addition of the above malonate (6.67 g, 0.02 mol). After refluxing sixty hours, the ethanol was evaporated and the material partitioned between water (pH 4) and ethyl acetate. The ethyl acetate was dried with $MgSO_4$ and evaporated to give a white gummy material. The material was then purified on silica prep plates using ethyl acetate/hexane (40/60) to give the purified barbituric acid.

5-(3-methylcyclohexyl)-5-(formylbutyl)-barbituric acid

Ozone was passed through a solution of the barbituric acid above (40 mg, 0.13 mmol) dissolved in methanol (20 ml) until the solution turned blue. Nitrogen was passed through the solution to remove excess ozone, and dimethyl sulfide (2 ml) was added. After stirring overnight, the solvents were removed to give the desired aldehyde as a white gummy material.

Example 6

Secbutyl dimethyl malonate

Sodium metal (6.9 g) was reacted with 120 ml of anhydrous methanol. To this solution was added 39.6 g (0.3 mol) of dimethylmalonate. The reaction mixture was refluxed, and 2-bromobutane (41.1 g, 0.3 mol) was added. After refluxing for 16 hours, the reaction mixture was cooled and methanol removed *in vacuo*. The product was extracted with ethyl ether and then washed with water and half saturated brine. Organics were dried over magnesium sulfate. After removal of ethyl ether by evaporation *in vacuo*, the crude oil was fractionally distilled to give 24 g of desired product (bp 95-105 °C/15mmHg).

5-secbutyl barbituric acid

110 ml of anhydrous ethanol was reacted with 7.72 g of sodium metal. Subsequently 17.6 g (0.1 mol) of dimethyl secbutylmalonate was added, followed (after about 5 minutes) by urea (6.72 g). The reaction mixture was refluxed for 19 hours and 60 ml of ethanol was distilled off. 200 ml of water was added to the residue followed by 20 mol of concentrated sulfuric acid. Precipitated crystals were filtered off. The product was then recrystallized from water to yield 8 g of colorless crystals.

5-secbutyl-5-(carbethoxy-1-propylene )barbituric acid

In a 2-neck 100 ml flask was placed 265 mg (6.625 mmol) of sodium hydride (60% oil suspension). The sodium hydride was washed with hexanes, and then 1219 mg of 5 secbutylbarbituric acid in 5 ml THF was added, followed by 50 ml of dimethylformamide. The reaction mixture was stirred at room temperature for 1.5 hours, and 912 ul of ethylbromocrotonate was added, followed by 900 mg of anhydrous potassium iodide. Reaction mixture was refluxed for 48 hours and subsequently rotoevaporated to dryness. The residue was extracted with ethyl acetate, washed with water, 5% sodium bisulfite, brine and then, dried over magnesium sulfate. The solvent was removed, and the crude material was purified by column chromatography on silica gel using ethyl acetate as an eluent. The yield was 78% of desired product.

5-secbutyl-5-(carboxy-1-propylene) barbituric acid

152 mg of 5-secbutyl-5-(carbethoxy-1-propylene) barbituric acid in 15 ml of concentrated hydrochloric acid was refluxed for 45 minutes. The reaction mixture was evaporated to dryness to yield the desired product as colorless crystals. (Yield 95%, mp 168-171 °C.)

Example 7

250 mg of 5-(1-methylbutyl)-5-carbethoxymethyl barbituric acid in 25 ml of concentrated hydrochloric acid was refluxed for 45 minutes. The reaction mixture was cooled, and the precipitated crystals were filtered off. Recrystallization from water gave 190 mg of colorless crystals (mp 238-240 °C.).

Example 8

200 mg of 5-(1-methylbutyl)-5-carbethoxypropyl barbituric acid was refluxed for 1 hour with 25 ml of concentrated hydrochloric acid. The reaction mixture was evaporated in vacuo and the solid residue was crystallized from water. Yield 120 mg of colorless crystals, mp 189-192 °C.

Example 9

Coupling of 5-secbutyl-5-carboxyl-propylene barbituric acid with aminomethylfluorescein

26.8 mg (0. 1 mmol) of 5-secbutyl-5-carboxy-1-propylene barbituric acid was dissolved in 0.3 ml of anhydrous DMF and to this solution was added 20 mg of dicyclohexylcarbodiimide dissolved in 0.3 ml of DMF, followed by 11.5 mg of N-hydroxysuccinimide in 0.3 ml of DMF. The reaction mixture was stirred at room temperature. After 30 minutes, 42 mg of aminomethylfluorescein was added. After 20 hours, the reaction mixture was rotoevaporated to dryness and purified by column chromatography on silica gel using ethyl acetate:acetic acid (100:0.2) as an eluent.

Example 10

Coupling of 5-(1-methylbutyl)-5-carboxyl-propylene) barbituric acid with aminomethyl fluorescein

This compound was prepared according to the procedure of Example 9, starting from 28.2 mg (0.1 mmol) of 5-(1-methylbutyl)-5-carboxycrotonoyl barbituric acid, 11.5 mg of N-hydroxysuccinimide, 20 mg of dicyclohexylcarbodiimide, and 42 mg of aminomethylfluorescein. The product was purified by preparative thin layer chromatography on silica gel using ethyl acetate:acetic acid (100:0.2) as an eluent.

Example 11

Coupling of 5-isopropyl-5-(carboxy 1-propylene) barbituric acid with aminomethylfluorescein

This compound was prepared according to the procedure of Example 9, starting with 21.4 mg of 5-ethyl-5-carboxymethyl barbituric acid, 11.5 mg of N-hydroxysuccinimide, 20 mg of dicyclohexylcarbodiimide and 42 mg of aminomethylfluorescein. The product was purified by preparative thin layer chromatography on silica gel using ethyl acetate:acetic acid (100:0.2) as an eluent.

Example 12

Coupling of 5-ethyl-5-carboxymethyl barbituric acid to aminomethylfluorescein

This compound was prepared according to the procedure of Example 9 starting with 21.4 of 5-ethyl-5-carboxymethyl barbituric acid, 11.5 mg of N-hydroxysuccinimide, 20 mg of dicyclohexylcarbodiimide and 42 mg of aminomethylfluorescein. The product was purified by chromatography on silica gel using ethyl acetate:acetic acid (100:0.2) as an eluent.

Example 13

Coupling of 5-secbutyl-5-carboxymethyl barbituric acid with aminomethylfluorescein

This compound was prepared according to the procedure of Example 9, starting with 24 mg of 5-secbutyl-5-carboxymethyl barbituric acid, 11.5 mg of N-hydroxysuccinimide, 20 mg of dicyclohexylcarbodiimide and 42 mg of aminomethylfluorescein. The product was purified by preparative thin layer chromatography (PTLC) on silica gel using ethyl acetate:acetic acid (100:0.2) as an eluent.

Example 14

Coupling of secbutyl-5-carboxyethyl barbituric acid with aminomethylfluorescein

This compound was prepared according to the procedure of Example 9, starting with 26 mg of 5-secbutyl-5-carboxyethylbarbituric acid, 11.5 mg of N-hydroxysuccinimide, 20 mg of dicyclohexyl carbodiimide and 42 mg of aminomethylfluorescein. The product was purified by PTLC on silica gel using ethyl acetate:acetic acid (100:0.2) as an eluent.

Example 15

Coupling of 5-secbutyl-5-carboxypropyl barbituric acid with amino-methylfluorescein

This compound was prepared according to the procedure of Example 9, starting with 28 mg of 5-secbutyl-5-carboxypropyl barbituric acid, 11.5 mg of N-hydroxysuccinimide, 20 mg of dicyclohexylcarbodiimide and 42 mg of aminomethylfluorescein. The product was purified by PTLC using ethyl acetate:acetic acid (100:0.2) as an eluent.

Example 16

Coupling of 5-secbutyl-5-carboxybutyl barbituric acid with aminomethylfluorescein

This compound was prepared according to the procedure of Example 9, starting with 29 mg of 5-secbutyl-5-carboxybutyl barbituric acid, 11.5 mg of N-hydroxysuccinimide, 20 mg of dicyclohexylcarbodiimide, and 42 mg of aminomethylfluorescein. The product was purified by PTLC using ethyl acetate:acetic acid, (100:0.2) as an eluent.

Example 17

Coupling of 5-1-(methylbutyl)-5-carboxy methyl barbituric acid with aminonethylfluorescein

This compound was prepared according to the procedure of Example 9 starting with 24 mg of 5-(1-methylbutyl)-5-carboxymethylbarbituric acid, 11.5 mg of N-hydroxysuccinimide, 20 mg of dicyclohexylcarbodiimide and 42 mg of aminomethylfluorescein. Product was purified by PTLC on silica gel using ethyl acetate - acetic acid, 100:0.2 as an eluent.

Example 18

Coupling of 5-(1-methylbutyl)-5-carboxyethyl barbituric acid with aminomethylfluorescein

This compound was prepared according to procedure of Example 9, starting with 25 mg of 5-(1-methylbutyl)-5-carboxyethyl barbituric acid, 11.5 mg of N-hydroxysuccinimide, 20 mg of dicyclohexylcarbodiimide and 42 mg of aminomethylfluorescein. The product was purified by PTLC on silica gel using ethyl acetate:acetic acid, (100:0.2) as an eluent.

Example 19

Coupling of 5-(1-methylbutyl)-5-carboxypropyl barbituric acid with aminomethylfluorescein

This compound was prepared according to the procedure of Example 9, starting with 26.5 mg of 5-(1-methylbutyl)-5-carboxypropyl barbituric acid, 11.5 mg of N-hydroxysuccinimide, 20 mg of dicyclohexylcarbodiimide and 42 mg of aminomethylfluorescein. The product was purified by PTLC on silica gel using ethyl acetate:acetic acid, (100:0.2) as an eluent.

Example 20

Coupling of 5-(1-methylbutyl)-5-carboxybutyl barbituric acid with aminomethylfluorescein

This compound was prepared according to the procedure of Example 9, starting with 28 mg of 5-(1-methylbutyl)-5-carboxybutyl barbituric acid, 11.5 mg of N-hydroxysuccinimide, 20 mg of dicyclohexylcarbodiimide and 42 mg of aminomethylfluorescein. The product was purified by PTLC on silica gel using ethyl acetate:acetic acid (100:0.2) as an eluent.

## Example 21

### Conjugation of 5-(1-methylbutyl)-5-carboxymethyl barbituric acid to fluoresceinamide (isomer)

To the 5-(1-methylbutyl)-5-carboxymethyl barbituric acid (58.3 mg) was added thionyl chloride (2 ml) and the solution refluxed for 1.5 hours. Excess of thionyl chloride was removed in vacuo and the oil cooled. To this was added a solution of fluoresceinamide (isomer 1) in dry pyridine (1 ml.). Chromatography of the material on silica prep plates using ethyl acetate:acetic acid (100:0.2) gave the desired tracer.

## Example 22

### Conjugation of 5-(3-methylcyclohexyl)-5-carboxybutyl barbituric acid

This compound was prepared according to the procedure of Example 9, starting from 21 mg of 5-(3-methylcyclohexyl)-5-carboxybutyl barbituric acid, 6.9 mg of N-hydroxysuccinimide, 19.9 of dicyclohexylcarbodiimide and 24 mg of aminomethylfluorescein.

## Example 23

### Conjugation of 5-(1-methylbutyl)-5-formyl methylbarbituric acid to aminomethylfluorescein

To a solution of 2 ml of water and 2 ml of methanol at pH 6 was added 5-(1-methylbutyl)-5-formylmethylbarbituric acid (141 mg, 0.58 mmol) and aminomethylfluorescein (210 mg, 0.58 mmol). The solution was completed by the dropwise addition of N,N-dimethylformamide. After stirring for 1 hour, sodium cyanoborohydride (38.8 mg) was added all at once. After stirring overnight, the solvents were evaporated and the material purified on reversed phase preparative plates (silica gel) using methanol/water/trifluoroacetic acid as an eluent.

## Claims

1. A compound comprising the structure:

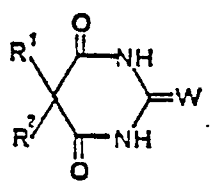

wherein:

W is oxygen or sulfur;
$R_1$ is alkyl, alkenyl, or alkynyl having a total of from 1 to 12 carbon atoms arranged in a straight or branched chain and containing up to two aliphatic or aromatic ring structures;
$R_2$ is $CH_2$-R-Z-Q;
Q is a poly(amino acid), a poly(amino acid) derivative or another immunologically active carrier; or fluorescein or a fluorescein derivative;
Z is NH, CO, CS, $SO_2$ or C=NH when Q is fluorescein or a fluorescein derivative and CO, O-CONH, N, NH, N=N, or $CH_2$ when Q is a poly(amino acid) or poly(amino acid) derivative or another immunologically active carrier; and
R is a linking group having a total of from 0 to 20 carbon atoms and heteroatoms arranged in a straight or branched chain and containing up to two ring structures; and including up to 7 heteroatoms when Q is a poly(amino acid), a poly(amino acid) derivative or other immunologically active carrier; up to 10 heteroatoms when Q is fluorescein or a fluorescein derivative and the linking group includes no ring structures; and up to 12 heteroatoms when Q is fluorescein or a fluorescein derivative and the linking group includes one or two ring structures.

2. The compound of Claim 1 wherein:
$R_1$ is alkyl having a total of from 2 to 7 carbon atoms arranged in a straight or branched chain and containing up to one aliphatic or aromatic ring structure;
Z is NH or CO when Q is fluorescein or a fluorescein derivative and $CH_2$ when Q is a poly(amino acid) or poly(amino acid) derivative or another immunologically active carrier; and
R is a linking group having a total of from 0 to 10 carbon atoms and heteroatoms arranged in a straight or branched chain and containing up to two ring structures; and including up to 4 heteroatoms when Q is a poly(amino acid), a poly(amino acid) derivative or other immunologically active carrier; up to 6 heteroatoms when Q is fluorescein or a fluorescein derivative and the linking group includes no ring structure; and up to 6 heteroatoms when Q is fluorescein or a fluorescein derivative and the linking group includes one or two aliphatic or aromatic ring structures.

3. The compound of Claim 1 wherein Q is bovine serum albumin.

4. The compound of Claim 2 wherein Q is an amino derivative of fluorescein.

5. An antibody to a compound according to Claim 1 when Q is a poly(amino acid) or a poly(amino acid) derivative.

6. A method for making a tracer comprising the step of coupling a precursor of the formula:

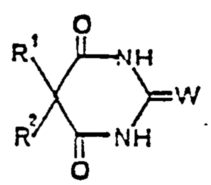

wherein:
W is oxygen or sulfur;
$R_1$ is alkyl, alkenyl or alkynyl having a total of from 1 to 12 carbon atoms arranged in a straight or branched chain and containing up to two aliphatic or aromatic ring structures;
$R_2$ is $CH_2$-R-Y;
Y is $NH_2$, COOH, COCl, $SO_3H$; $SO_2Cl$, SH, CHO, CN, OH, or I; and
R is a linking group including up to 10 heteroatoms, having a total of from 0 to 20 carbon atoms and heteroatoms arranged in a straight or branched chain and containing up to two aliphatic or aromatic ring structures;
with fluorescein or a derivative of fluorescein.

7. The method of Claim 6 wherein:
$R_1$ is alkyl having 4 or 5 carbon atoms arranged in a branched chain and containing no ring structures;
Y is $NH_2$ or COOH; and
R is a linking group including up to 3 heteroatoms, having a total of from 3 to 5 carbon atoms and heteroatoms arranged in a straight or branched chain and containing no ring structure.

8. The method of Claim 7 wherein the fluorescein derivative is aminomethylfluorescein.

9. A process for detecting the presence of barbiturates in biological fluids, comprising the steps of:
(a) containing a sample with a barbiturate antiserum and a tracer compound according to Claim 1 capable of producing a detectable fluorescence polarization response o the presence of the barbiturate

antiserum;

(b) passing plane polarized light through the resulting solution from step (a) to obtain a fluorescence polarization response; ad

(c) detecting the fluorescence polarization response of the solution of step (b) as a measure of the presence of barbiturate in the sample.

10. The process of Claim 9 wherein step (a) the sample is additionally contacted with riboflavin binding protein.

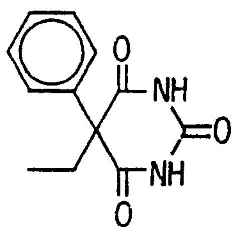

FIG. 1-1

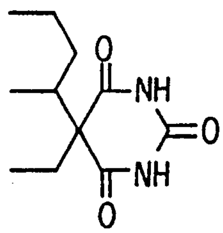

FIG. 1-2

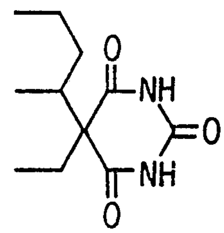

FIG. 1-3

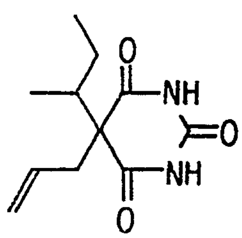

FIG. 1-4

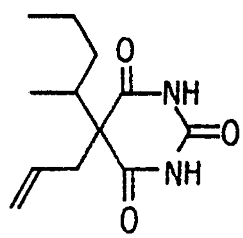

FIG. 1-5

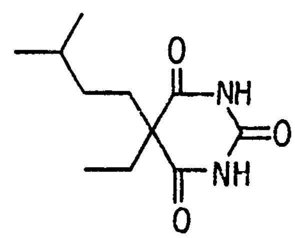

FIG. 1-6

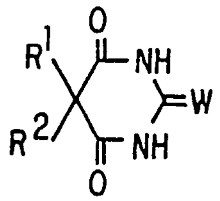

W = O,S

$R^2 = -CH_2-R-Z-Q$

FIG. 2

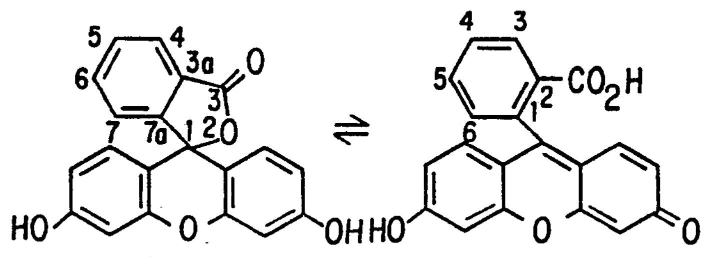

LACTONE ACID

FIG. 3

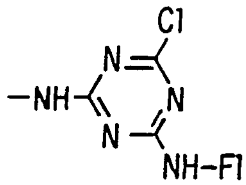

FIG. 4-1

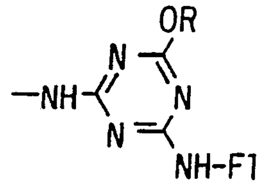

FIG. 4-2

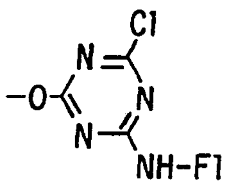

FIG. 4-3

-NH-CO-Fl

FIG. 4-4

-CO-NH-Fl

FIG. 4-5

-C(NH)-NH-Fl

FIG. 4-6

-NH-CO-NH-Fl

FIG. 4-7

-NH-CS-NH-Fl

FIG. 4-8

-O-CO-NH-Fl

FIG. 4-9

-O-CS-NH-Fl

FIG. 4-10

$-SO_2-NH-Fl$

FIG. 4-11

$-O-CO-NH-SO_2-NHFl$

FIG. 4-12

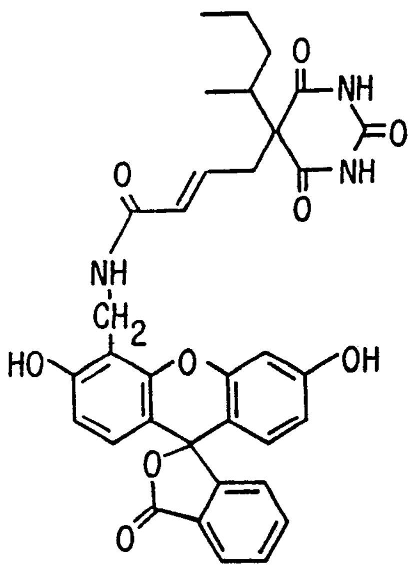

FIG. 5

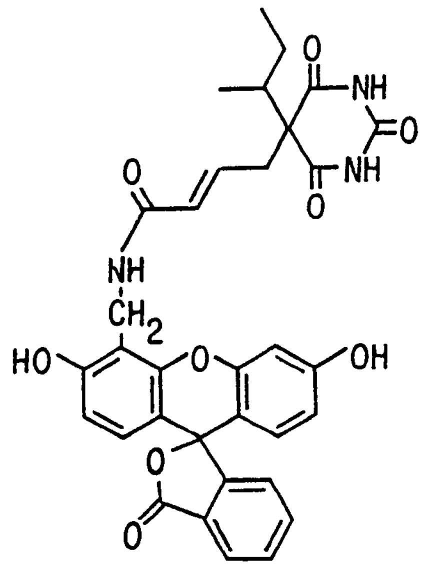

FIG. 6

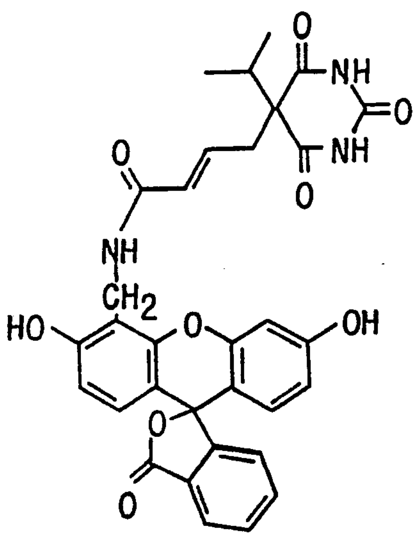

FIG. 7

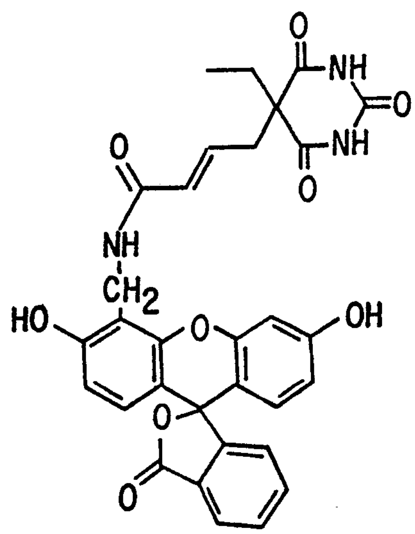

FIG. 8

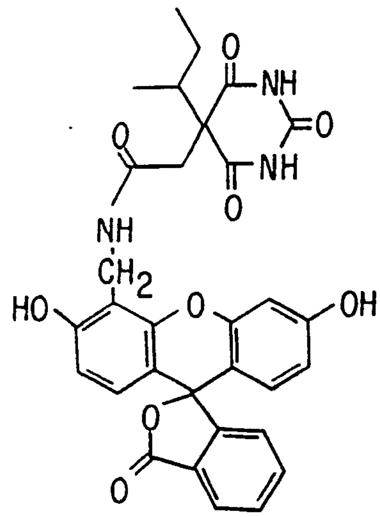

FIG. 9

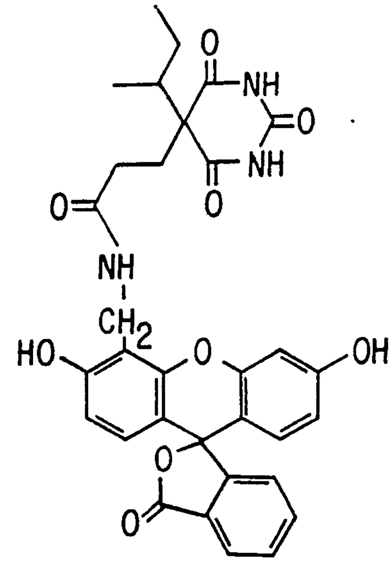

FIG. 10

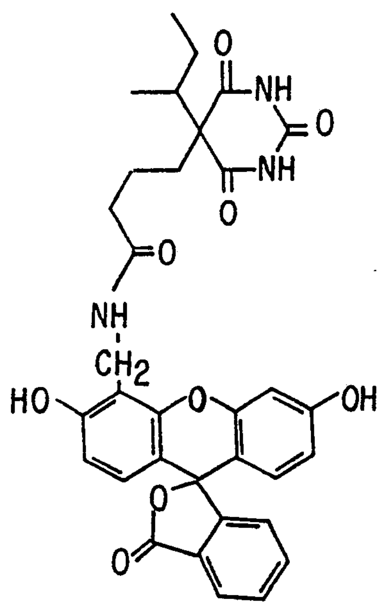

FIG. 11

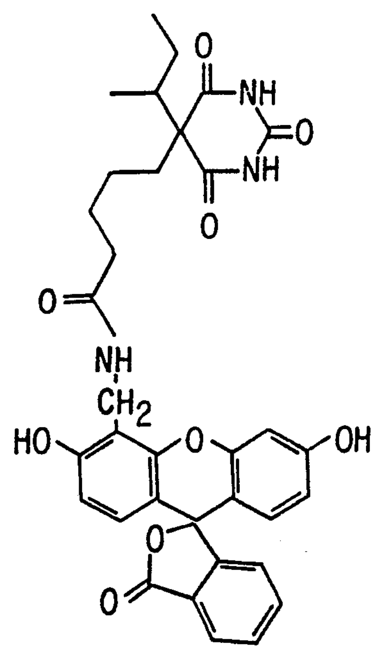

FIG. 12

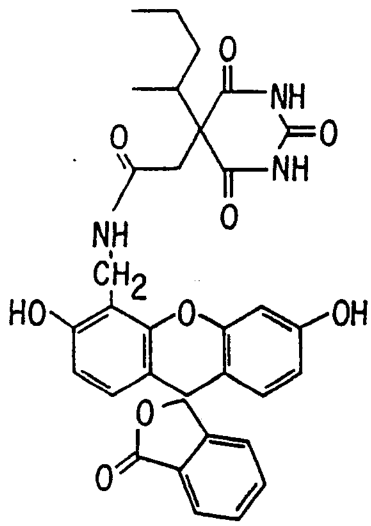

FIG. 13

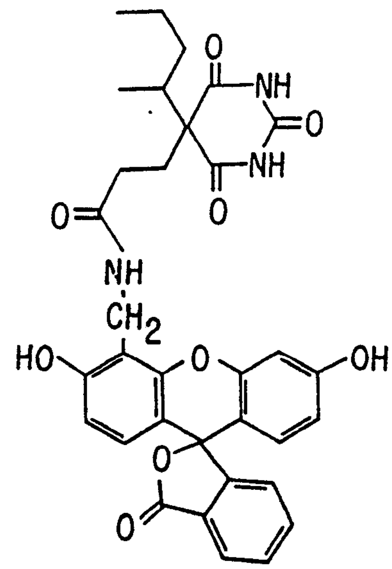

FIG. 14

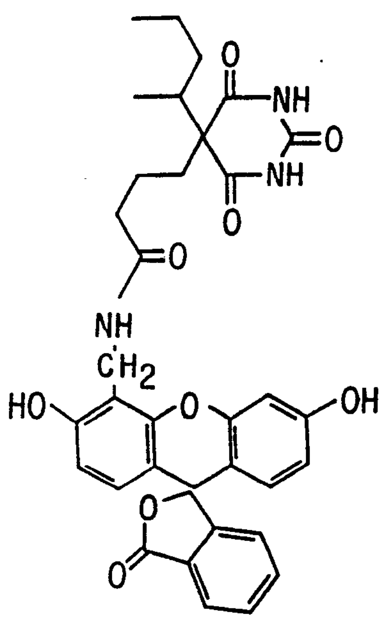

FIG. 15

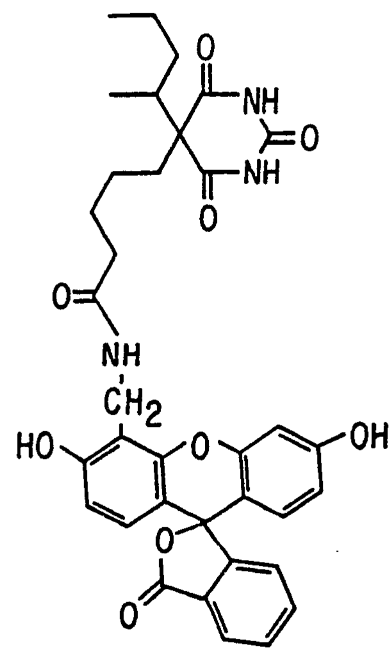

FIG. 16

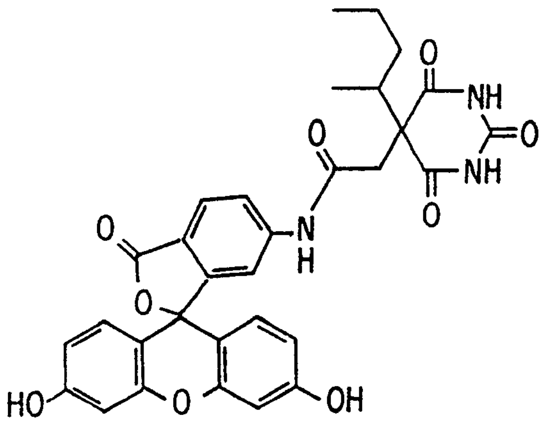

FIG. 17

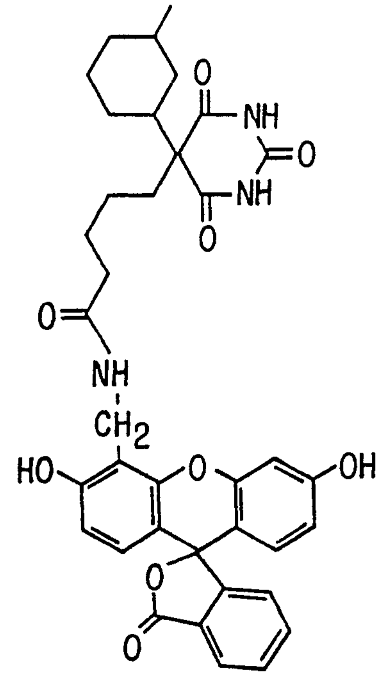

FIG. 18

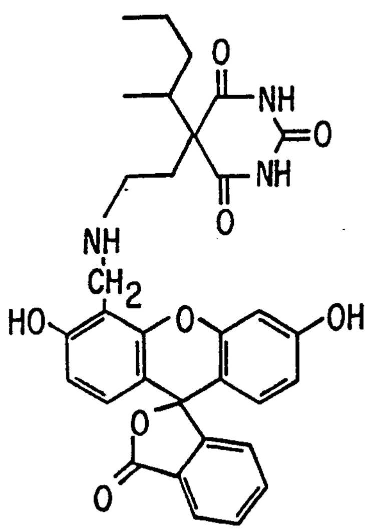

FIG. 19

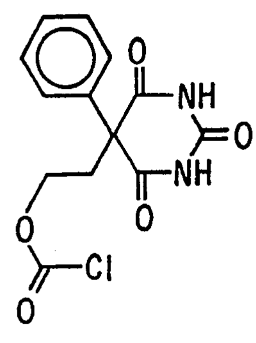

FIG. 20

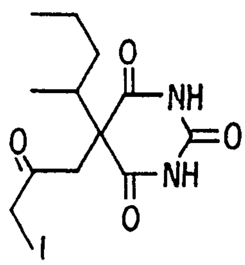

FIG. 21

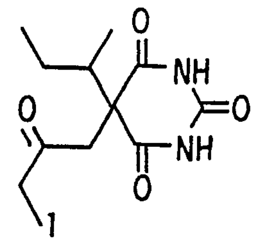

FIG. 22

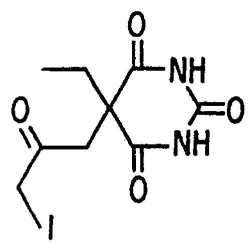

FIG. 23

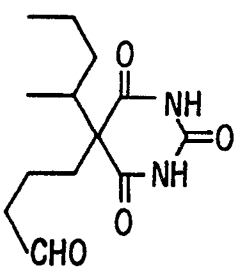

FIG. 24

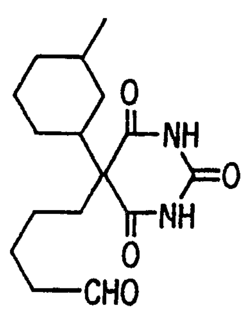

FIG. 25

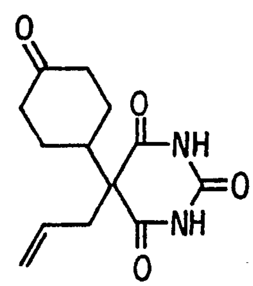

FIG. 26